# EUROPEAN PATENT APPLICATION

(11) **EP 4 744 699 A2**
(43) Date of publication of application: **20.05.2026**
(21) Application number: 26169942.5
(22) Date of filing: 14.12.2021
(51) Int. Cl.: A61M 5/32

(54) **FLUID DELIVERY SYSTEM WITH NEEDLE ASSEMBLY**

(30) Priority: 17.12.2020 US 202063126611 P
(62) Divisional of application: 21840332.7
(71) Applicant: Eli Lilly and Company, Indianapolis, IN 46285 (US)
(72) Inventor: GUNAY, Murat, Indianapolis, 46206-6288 (US); JUDSON, Jared Alden, Indianapolis, 46206-6288 (US); PERKINS, Russell Wayne, Indianapolis, 46206-6288 (US); SCHAFF, Anthony Lawrence, Indianapolis, 46206-6288 (US)
(74) Representative: J A Kemp LLP

(57) **Abstract**

A needle assembly comprising: a housing configured to interface with a surface; a first needle supported by a first needle support positioned at least partially within the housing; a second needle supported by a second needle support positioned at least partially within the housing; a driving member supported by the housing and configured to engage with the first needle support and the second needle support and to move the first and second needle supports relative to the housing; a first fluid coupler in fluid communication with the first needle; and a second fluid coupler in fluid communication with the second needle, wherein the first and second fluid couplers fluidly couple the first and second needles when the first and second fluid couplers are aligned.

## Description

### FIELD OF THE DISCLOSURE

The present disclosure relates to a needle assembly to deliver a fluid. More specifically, the present disclosure relates to an extendable and retractable needle assembly configured to deliver a fluid through a surface.

### BACKGROUND OF THE DISCLOSURE

Conventional injection devices are often used to drive a needle through a surface, for example in the injection of a drug, removing a fluid from a sealed container such as a vial, sampling within chemical instrumentation, and so on. Considering the example of injecting a patient with a drug, it is sometimes advantageous for the drug to be administered without the presence of a medical professional (e.g. when taken frequently). It may be a challenge to ensure that the needle is maintained in a sterile environment prior to injection, as well to ensure that the drug is administered safely and efficiently. Furthermore, some patients are uncomfortable with seeing or directly handling needles.

### SUMMARY

A needle assembly configured to be part of a drug delivery device comprises a number of extendable and retractable needles. The needles are fluidly coupled when extended, and fluidly decoupled when retracted. When the needles are extended, a first needle pierces a patient's skin, and a second needle pierces a septum of a drug cartridge. Once the drug has been delivered, the needles may then retract within a needle assembly housing. The needle assembly may also be part of a partially disposable drug delivery device, and may be used with multiple different drug cartridges. Multiple embodiments of the needle assembly are disclosed.

In one embodiment, a drug delivery device including a cassette, a cartridge within the cassette configured to hold a medication, and a needle assembly coupled to the cassette. The needle assembly includes a housing with a first end and a second end configured to couple to the cassette. A needle mechanism is supported by the housing, and includes a first needle and a second needle. The needle mechanism is movable from a first configuration to a second configuration. In the first configuration, the needle mechanism is positioned fully within the housing and, in the second configuration, the first needle extends beyond the first end and the second needle extends beyond the second end. A first fluid coupler and a second fluid coupler is within the needle mechanism and fluidly coupled to the first needle and the second needle respectively. The first and second fluid couplers fluidly disconnect the first and second needles when the needle mechanism is in the first configuration and fluidly couple the first and second needles when the needle mechanism is in the second configuration. In another embodiment, a method of operation of a device comprising the steps of: Positioning the device, the delivery device comprising a cassette, a drug cartridge within the cassette, and movable needle assembly coupled to the cartridge. Activating a driving member of the needle assembly, the driving member moving a first needle from the needle assembly in a first direction from a retracted configuration to an axially extended configuration, and moving a second needle from the needle assembly in a second direction towards the drug cartridge. Fluidly coupling the first needle and the second needle. Activating a delivery mechanism to drive a medication from the drug cartridge through the needle assembly. Reactivating the driving member to move the first needle in the second direction from the axially extended configuration to the retracted configuration, and to move the second needle in the first direction away from the septum.

In another embodiment, a needle assembly comprising a housing configured to interface with a surface. A first needle is supported by a first needle support positioned at least partially within the housing. A second needle is supported by a second needle support positioned at least partially within the housing. A driving member is supported by the housing and configured to engage with the first needle support and the second needle support and to move the first and second needle supports relative to the housing. A first fluid coupler in fluid communication with the first needle, and a second fluid coupler in fluid communication with the second needle. The first and second fluid couplers fluidly couple the first and second needles when the first and second fluid couplers are aligned.

In another embodiment, a needle assembly includes a housing with a first end and a second end. A plurality of needle mechanisms is supported by the housing. Each of the needle mechanisms includes a first needle support configured to move in a first direction, and a second needle support configured to move in a second direction. The first fluid coupler is coupled to the first needle support, and a second fluid coupler is coupled to the first needle support. The first fluid coupler and the second fluid coupler provide fluid communication between the first needle and the second needle when the first and second fluid couplers are aligned. A rotating assembly is configured to rotate the needle mechanisms within the housing.

### BRIEF DESCRIPTION OF THE DRAWINGS

The above-mentioned and other features and advantages of this disclosure, and the manner of attaining them, will become more apparent and the invention itself will be better understood by reference to the following description of embodiments of the invention taken in conjunction with the accompanying drawings, wherein:
FIG. 1 is a simplified, cross-sectional view of an exemplary drug delivery device;
FIG. 2 is a simplified, cross-sectional view of another exemplary drug device with a detachable cassette and needle assembly;
FIG. 3 is a simplified, cross-sectional view of yet another exemplary drug delivery device with a detachable needle assembly;
FIG. 4 is a side elevational view of an exemplary drug delivery device having a cassette and a driving module;
FIG. 5 is a vertical cross section of the device of FIG. 4;
FIG. 6 is a cross section taken along line B-B of FIG. 5;
FIG. 7 is a cross section taken along line C-C of FIG. 5;
FIGS. 8-10 are exploded views of the device of FIG. 4 showing the driving module and the cassette separated;
FIG. 11 is a perspective view of another embodiment of an exemplary drug delivery device having a cassette and a needle assembly;
FIG. 12 is an exploded view of the drug delivery device of FIG. 11;
FIG. 13 is a perspective view of a cartridge from the device of FIG. 11;
FIG. 14 is a perspective view of a driving mechanism of the device of FIG. 11;
FIG. 15 is a simplified, diagrammatic view of the driving mechanism of FIG. 14;
FIG. 16 is a diagrammatic view of a control system for the driving mechanism of FIG. 14;
FIG. 17 is a perspective view of an exemplary needle assembly;
FIG. 18 is an exploded view of the needle assembly of FIG. 17;
FIG. 19 is a cross-sectional view of the needle assembly of FIG. 17 in a first configuration;
FIGS. 20 and 21 are perspective views of a needle mechanism of the needle assembly of FIG. 17 in the first configuration;
FIGS. 22 and 23 are perspective views of a driving member of the needle mechanism of FIGS. 20 and 21;
FIGS. 24 and 25 are side views of the needle mechanism of FIGS. 20 and 21 showing a motion of the needle mechanism between the first configuration of FIG. 24 and the second configuration of FIG. 25;
FIG. 26 is another exemplary embodiment of a needle assembly;
FIG. 27 is an exploded view of the needle assembly of FIG. 26;
FIG. 28 is a perspective view of the needle mechanisms of the needle assembly of FIG. 27; and
FIG. 29 is a diagrammatic view of an exemplary wireless communication system for use with a drug delivery device.

Corresponding reference characters indicate corresponding parts throughout the several views. The exemplifications set out herein illustrate exemplary embodiments of the invention and such exemplifications are not to be construed as limiting the scope of the invention in any manner.

### DETAILED DESCRIPTION

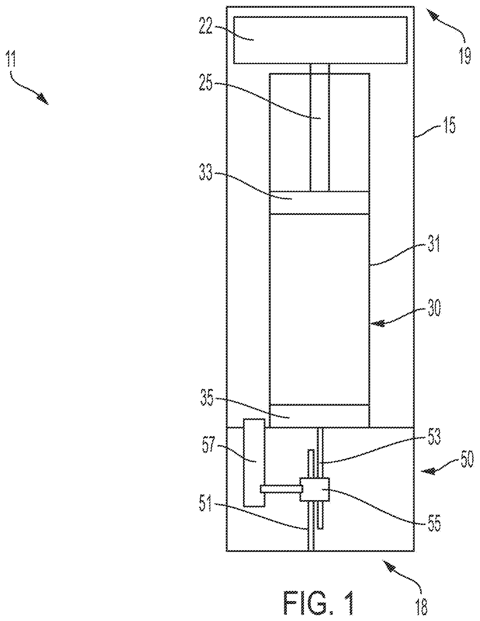

Exemplary drug delivery devices 11, 12, 13, 126, and 1000 are illustrated in FIGS. 1-4 and 11. Each of the shown devices 11, 12, 13, 126, and 1000 is a reusable pen-shaped medication injection device which may be manually handled by a user (e.g., a patient, a caregiver, or a healthcare professional) to deliver a medication to a patient. In certain embodiments, the user may selectively set a dose and then to inject that set dose into the patient. Devices 11, 12, 13, 126, and 1000 are intended to be illustrative and not limiting as the needle assemblies described further below may be used in other differently configured devices.

The medication may be any type that may be delivered by such a device 11, 12, 13, 126, and 1000. The medication may be in fluid form or any other suitable form. The medication includes one or more therapeutic agents including but not limited to insulins, insulin analogs such as insulin lispro or insulin glargine, insulin derivatives, GLP-1 receptor agonists such as dulaglutide or liraglutide, glucagon, glucagon analogs, glucagon derivatives, gastric inhibitory polypeptide (GIP), GIP analogs, GIP derivatives, dual agents of any combination above, such as, for example, GIP/GLP-1 receptor agonist, oxyntomodulin analogs, oxyntomodulin derivatives, therapeutic antibodies and any therapeutic agent that is capable of delivery by the device 11, 12, 13, 126, and 1000. The medication as used in the device 11, 12, 13, 126, and 1000 may be formulated with one or more excipients.

Referring first to FIG. 1, an exemplary embodiment of a drug delivery device 11 is shown. Drug delivery device 11 extends from a proximal end 19 to a distal end 18, and comprises a housing 15, a first motor 22, a driving system 25, a cartridge 30 configured to retain the medication, and a needle assembly 50. In the illustrated embodiment, drug delivery device 11 is configured to be positioned with distal end 18 near a patient's skin, such that needle assembly 50 may deliver the medication from within cartridge 30 to the patient. Cartridge 30 may also be described as a cassette, or a syringe, and is generally configured to contain and at least partially deliver the medication.

Cartridge 30 additionally comprises a fluid housing or barrel 31, a plunger or stopper 33, and a septum 35. The medication is retained within fluid housing 31 by stopper 33. The activation of first motor 22 actuates driving system 25 to drive stopper 33 downward to push the medication towards septum 35 and ultimately through needle assembly 50. First motor 22 may be controlled by a motor controller (not shown) to adjust the force applied to driving system 25 and/or the rate that driving system 25 is actuated.

Needle assembly 50 additionally comprises a first needle 51, a second needle 53, a needle driving mechanism 55 coupled to the first and second needles 51, 53, and an optional second motor 57 configured to actuate needle driving mechanism 55. Activation of second motor 57 actuates needle driving mechanism 55 and drives first needle 51 towards distal end 18 and drives second needle 53 towards proximal end 19 into an extended configuration whereby first needle 51 pierces at least the patient's skin, and second needle 53 pierces septum 35. In some embodiments, drug delivery device 11 only comprises first motor 22 and does not comprise the second motor 57. In such embodiments, first motor 22 may be coupled or linked to needle driving mechanism 55 such that activation of first motor 22 may additionally actuate needle driving mechanism 55. As will be described in more detail later, first needle 51 and second needle 53 are fluidly coupled together to allow flow of the medication from cartridge 30, through second needle 53, through first needle 51, and ultimately to the patient.

Drug delivery device 11 is configured as a singular device and may also be configured to be disposable after use. In the illustrated embodiment, cartridge 30 and needle assembly 50 are fixedly coupled to and/or an integral part of drug delivery device 11. Cartridge 30 may contain a single dose of the medication to be delivered or may contain the medication to be delivered in multiple doses/injections. In embodiments where cartridge 30 comprises multiple doses of the medication, first motor 22 may be configured to only push out a portion of the medication within cartridge 30 for each dose, and needle assembly 50 may comprise multiple sets of needles to inject the medication into a patient.

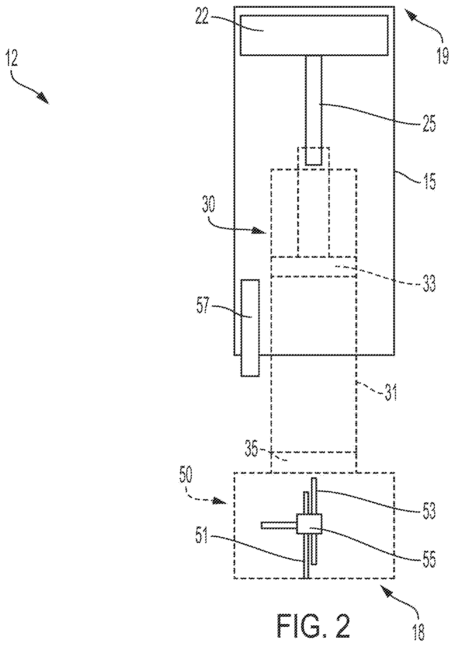

Referring now to FIG. 2, another embodiment of a drug delivery device 12 is shown. Drug delivery device 12 functions similar to drug delivery device 11 of FIG. 1 and comprises similar components. However, drug delivery device 12 differs from drug delivery device 11 in that cartridge 30 and needle assembly 50 are removable from housing 15. Needle assembly 50 and cartridge 30 may be separate components from one another or may be one single component. In the illustrated embodiment, housing 15, first motor 22, driving system 25, and second motor 57 may all be reusable, and cartridge 30 and needle assembly 50 may be disposable. In such an embodiment, a user may insert, use, and remove multiple instances of cartridge 30 and needle assembly 50, either together or separately. As was the case with drug delivery device 11, cartridge 30 of drug delivery device 12 may still comprise the medication for a single dose or multiple doses. Cartridge 30 and needle assembly 50 may couple to housing 15 of drug delivery device 12 through any suitable coupling mechanism, including but not limited to threading, bayonet couplers, screws, rivets, snaps, ball-detent mechanisms, spring systems, pins, magnets, friction fitting, adhesives, or any other suitable coupling device, and combinations thereof. In embodiments where cartridge 30 and needle assembly 50 are separate components, any suitable coupling device such as those mentioned above may additionally be used to couple cartridge 30 and needle assembly 50. In the illustrated embodiment, second motor 57 is coupled to housing 15, and is not shown as part of needle assembly 50 or cartridge 30, but instead couples to needle assembly 50 when needle assembly 50 is coupled to housing 15. In other embodiments, second motor 57 may be part of needle assembly 50 and may be couplable and decouplable from housing 15 with needle assembly 50.

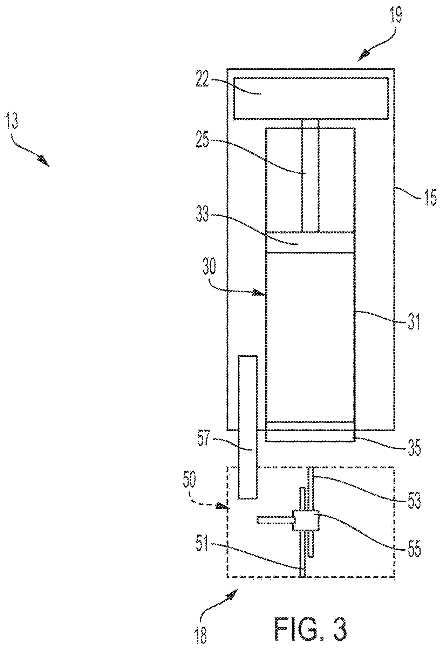

Referring now to FIG. 3, another embodiment of a drug delivery device 13 is shown. Drug delivery device 13 is similar to drug delivery device 11 of FIG. 1 and drug delivery device 12 of FIG. 2, except that cartridge 30 is fixedly coupled to housing 15, and needle assembly 50 is removably coupled to housing 15. Needle assembly 50 may be disposable while cartridge 30 and/or housing 15 may be reusable. In the illustrated embodiment, housing 15 and cartridge 30 may be used multiple times with different instances of needle assembly 50. In some embodiments, cartridge 30 may be refillable with the medication. As mentioned before with respect to drug delivery device 12, needle assembly 50 may be coupled to housing 15 through any suitable coupling device or system. Additionally, second motor 57 may be part of housing 15 or needle assembly 50. Additional embodiments, configurations, and details of drug delivery devices and their components will be discussed below.

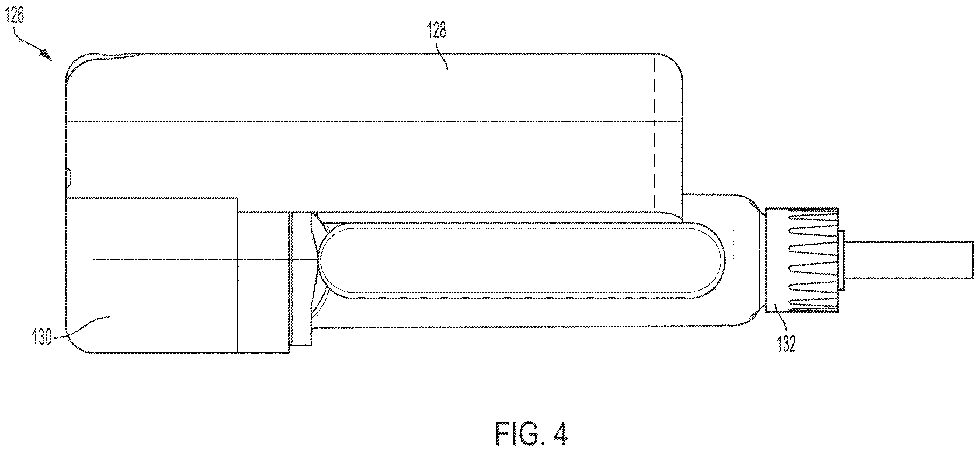

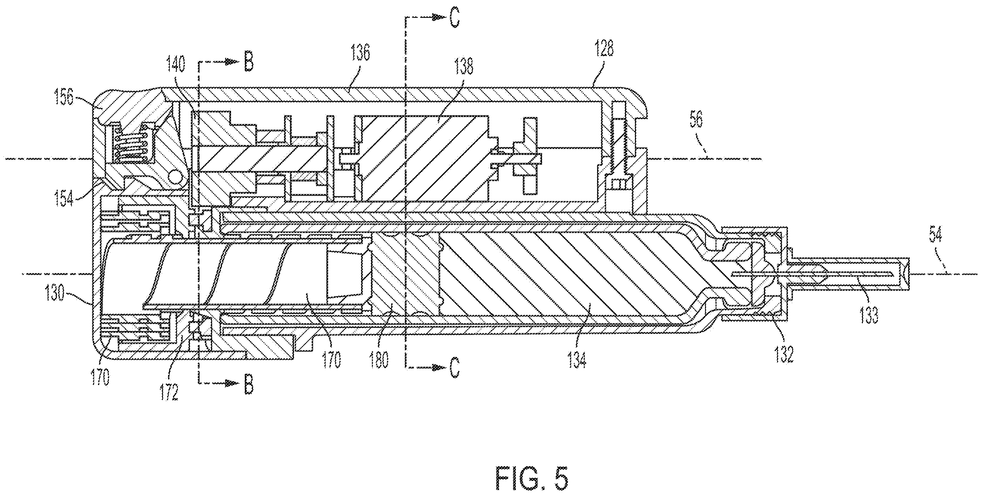

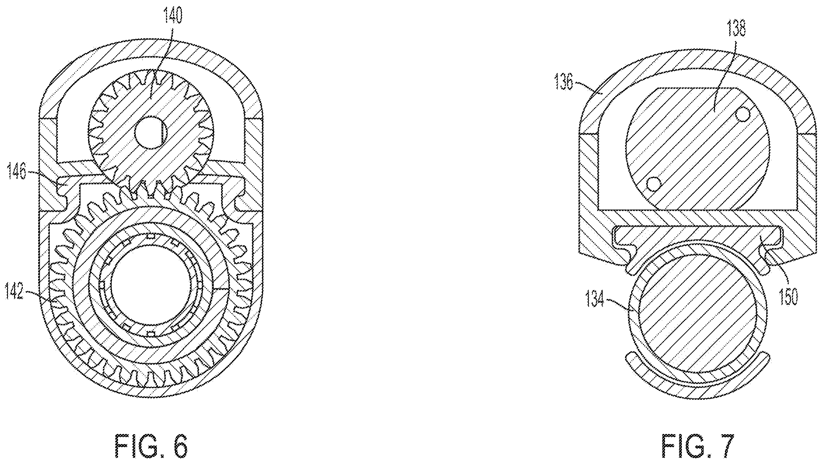

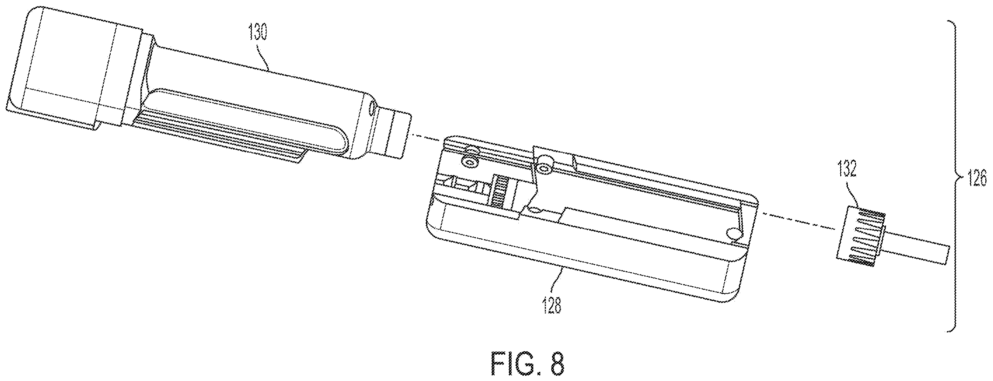

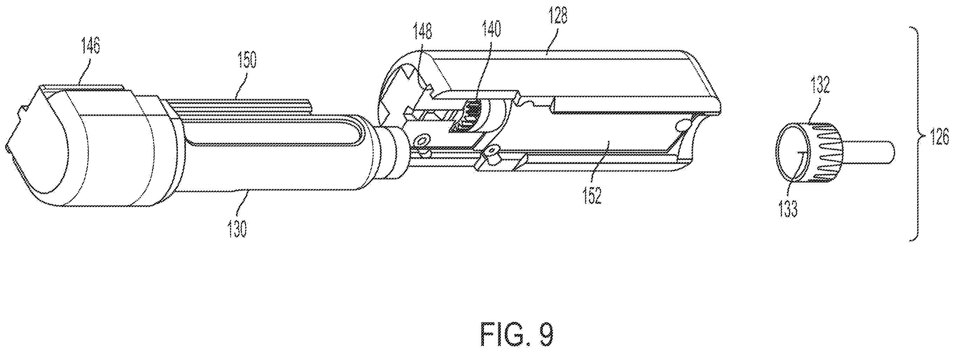

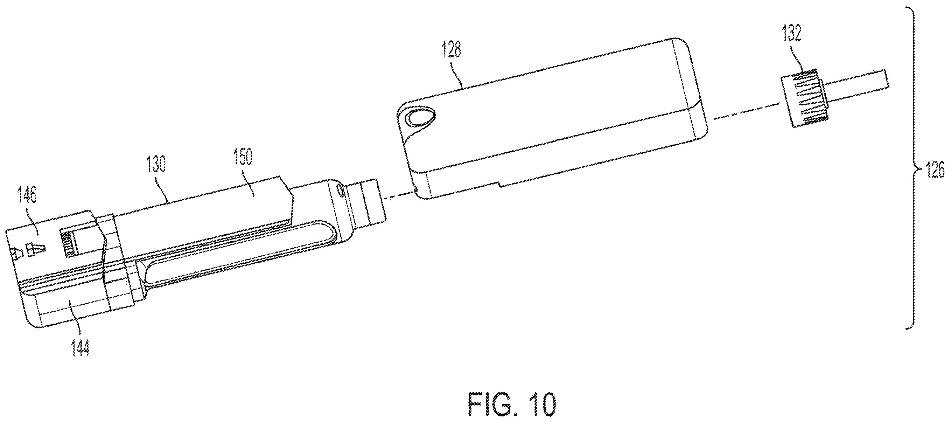

Referring first to FIGS. 4-10, another embodiment of a drug delivery device 126 is provided. Drug delivery device 126 includes a reusable driving module 128 and a cassette 130. A needle assembly 132 is securable to the threaded distal end of a medication-containing cartridge 134. Reusable driving module 128 includes a housing 136 within which an electric motor 138 is mounted. A driving gear 140 is coupled to and driven by the output shaft of electric motor 138. A portion of driving gear 140 projects outwardly from housing 136 whereby it can engage a driven gear 142 within cassette 130 when cassette 130 is attached to reusable driving module 128.

Cassette 130 includes a housing 144 that defines a first T-shaped projection 146. Housing 136 defines a corresponding first T-shaped slot 148 which receives the first T-shaped projection 146. Cassette 130 also defines a second T-shaped projection 150 which is received by a second T-shaped slot 152 on housing 136. When the T-shaped projections 146, 150 are slid into the T-shaped slots 148, 152, a spring biased, pivoting latch member 154 on reusable driving module 128 engages a projecting lip on the cassette to prevent the cassette 130 from sliding out of engagement. Button 156 is depressed to disengage latch member 154. The drug delivery device 126 further comprises a drive ribbon 170 defining a first primary axis 54 and a drive member mechanism that defines a secondary parallel axis 56. The drive ribbon 170 is disposed within the cassette 130, and a trust member 172 controls the axial extension of drive ribbon 170.

The drug delivery device 126 may be used to administer the above-described medication to the patient through needle assembly 132. In the illustrated embodiment, the cassette 130 is configured to be decoupled from the reusable driving module 128. Generally, the drive ribbon 170 is configured to extend a needle 133 outward from needle assembly 132 along the first primary axis 54 and/or drive a drug through said needle 133 by pushing a stopper 180 through the cartridge 134. A number of examples of drug delivery device 126 and variations thereof are disclosed in PCT Publication No. WO 2019/112886, published on June 13, 2019, the entire disclosure of which is expressly incorporated by reference herein.

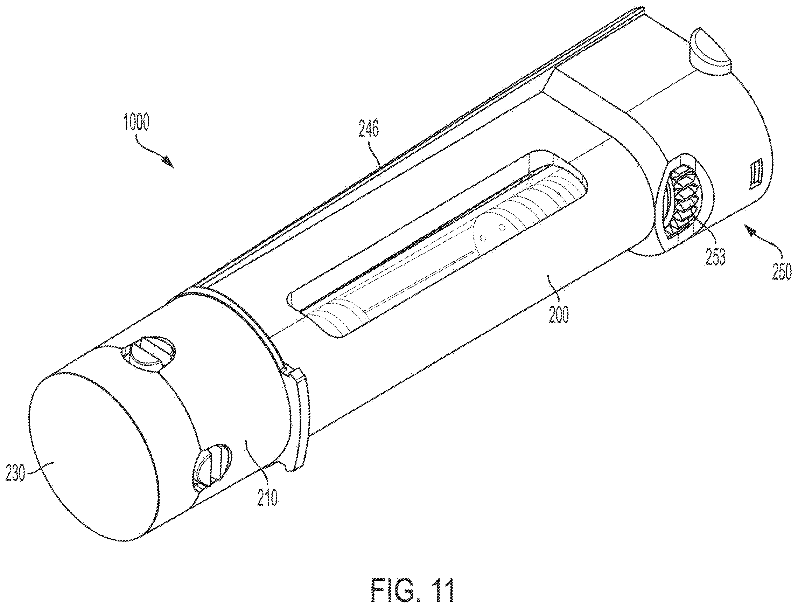

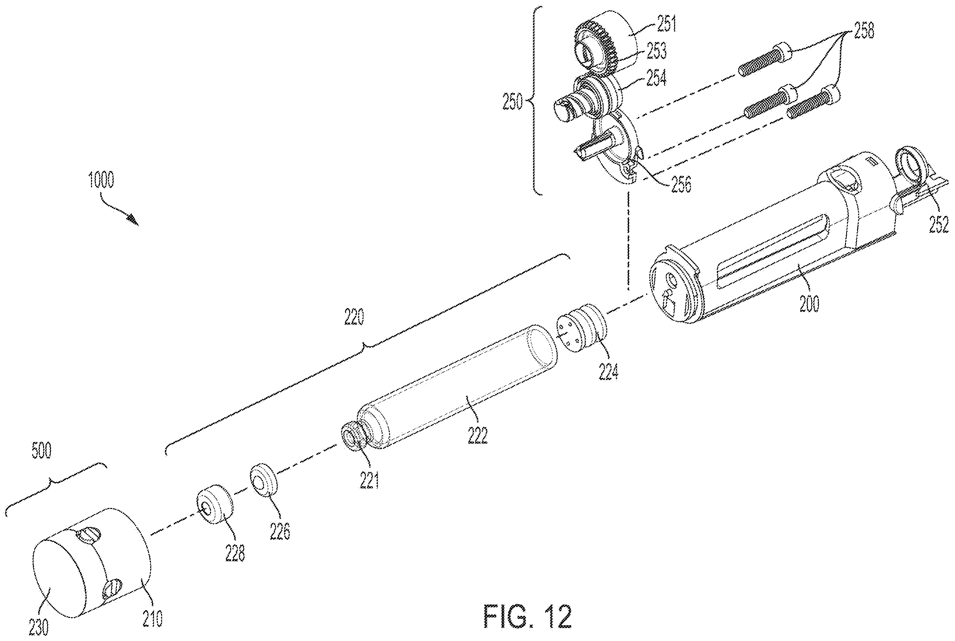

Referring now to FIGS. 11 and 12, another exemplary drug delivery device 1000 is shown. Drug delivery device 1000 comprises a cassette 200 configured for use with the driving module 128 of FIGS. 4-10 or another suitable driving module. The illustrative cassette 200 includes a driving system 250, a cartridge 220, and a needle assembly 500 having a seal 230. The illustrative cassette 200 also includes one or more mating elements such as T-shaped projections 246 (similar to T-shaped projections 146, 150) configured to interact with the corresponding driving module.

The cartridge 220 is positioned within cassette 200 and is configured to hold the above-described medication. The needle assembly 500 is generally configured to interact with a surface, for example a patient's skin, and to pierce a first needle (shown elsewhere) through the surface. Once the first needle has pierced the surface, the driving system 250 will drive the medication from within cartridge 220 through the needle assembly 500 and into or through the surface. Needle assembly 500 is then configured to retract the needle within the needle assembly 400. While the needle assembly 500 is shown in the illustrated embodiment of FIGS. 11 and 12, it should be understood that any embodiment of a needle assembly as disclosed herein may be used within drug delivery device 1000.

Like drug delivery device 126, in the illustrated embodiment the cassette 200 is configured to be coupled and decoupled from an element of drug delivery device 1000, in this case needle assembly 500. In the illustrated embodiment, needle assembly 500 is coupled to cassette 200 through a needle assembly coupler 210, but in other embodiments needle assembly 500 may be configured to couple directly to cassette 200 without the need for an additional coupler. Needle assembly 400 may be permanently or removably coupled to cassette 200 (as discussed above regarding FIGS 1-3), and may couple to cassette 200 through any suitable coupling devices or systems, including but not limited to threading, bayonet couplers, screws, rivets, snaps, ball-detent mechanisms, spring systems, pins, magnets, friction fitting, adhesives, or any other suitable coupling device and combinations thereof. In other embodiments, needle assembly 400 may be an integral part of cassette 200. Needle assembly 500 may be a disposable component or may be used multiple times with different cassettes 200. Cassette 200 may be configured to be entirely or partially disposable. For example, cassette 200 and driving system 250 may be reusable, and cartridge 220 may be disposable. In such an embodiment, cartridge 220 may be couplable and decouplable from cassette 200 and/or driving system 250. In another embodiment, each of cassette 200, driving system 250, and cartridge 220 may be disposable, or the entirety of drug delivery device 1000 may be disposable.

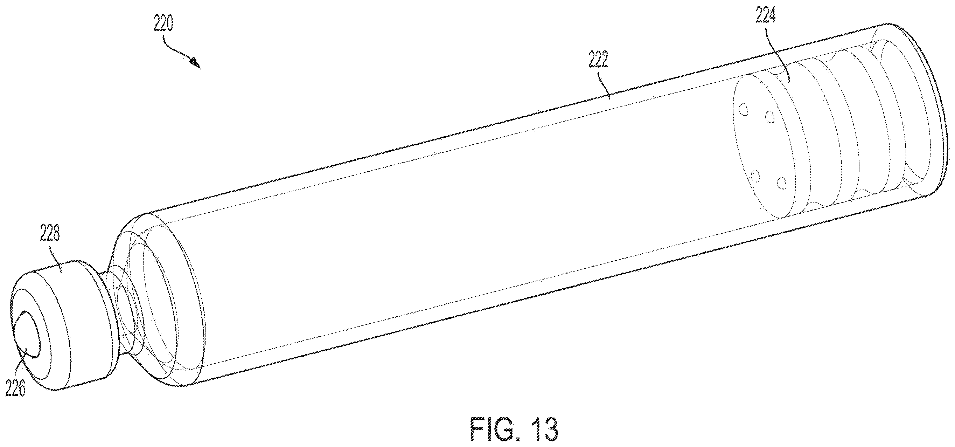

Referring now to FIGS. 12 and 13, cartridge 220 comprises a fluid housing 222, a stopper 224, a septum 226, and a septum seal 228. Fluid housing 222 is configured to hold a volume of the medication, which is retained within fluid housing 222 by the stopper 224 and the septum 226. The septum 226 is configured to be pierced by a septum needle (shown elsewhere) to allow for the fluid to flow through the needle that has pierced septum 226. The stopper 224 or piston is configured to slide along the interior of fluid housing 222 to increase the pressure within fluid housing 222 and drive the fluid through the septum needle within septum 226. In the illustrated embodiment, stopper 224 is driven by the driving system 250, but in other embodiments the stopper 224 may be driven by any mechanical, electrical, or human-activated drive system. Common drive systems include drive screws, linear drives activated, such as, for example, springs or electric motors, flexible rack/pinion drive, etc. The septum seal 228 is configured to retain septum 226 on a hub 221 of fluid housing 222. The cartridge 220 may be assembled and filled by any standard method as is known in the art. For example, septum 226 may be positioned against hub 221 and secured to hub 221 through septum seal 228. Fluid housing 222 may then be filled with a fluid and sealed with stopper 224. In an exemplary embodiment, each of the components of cartridge 220 is sterilized before use. Fluid housing 222 may be composed of glass, plastic, or any other material configured to retain a fluid. Septum seal 228, septum 226, and stopper 224 may be composed of an elastomer, plastic, or any other polymer or composite.

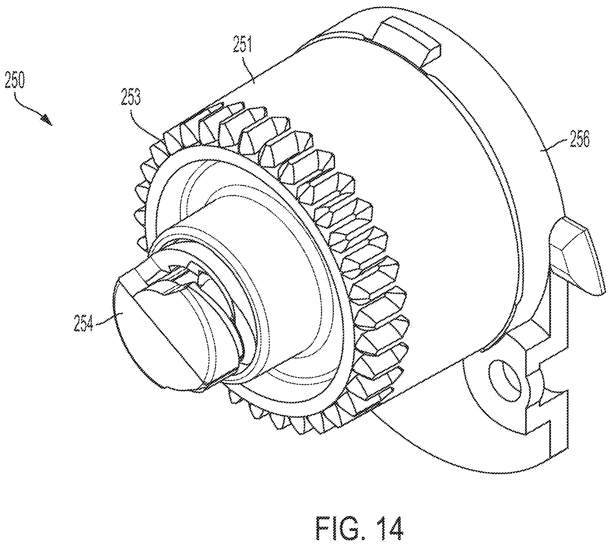

Referring now to FIGS. 12 and 14, an exemplary embodiment of the driving system 250 comprises a support 256, a collar 252, an activating member 251 having a driven gear 253, a driving member 254, and fasteners 258. The driving system 250 may also be referred to as a delivery mechanism. The driven gear 253 of the activating member 251 is exposed through the cassette 200 and configured to be driven by a user or by an external mechanism (e.g. a motor such as first motor 22 shown in FIGS. 1-3) (similar to driven gear 142 of FIG. 1) to then engage driving member 254 and cause driving member 254 to drive stopper 224 within cartridge 220. In the illustrated embodiment, driving member 254 is a drive screw and is threaded to be driven by driven gear 253. Rotation of driven gear 253 causes axial movement of driving member 254 towards or away from stopper 224. In other embodiments, other driving systems may be used such as a rack/pinion driving system. The activating member 251 and driving member 254 are both supported by support 256, which is coupled to cassette 200 through collar 252 and fasteners 258. In the illustrated embodiment, fasteners 258 are screws, but in other embodiments may be any fastening device such as rivets, nails, clips, clamps, snaps, welds, adhesives, or other fastening devices or systems. Other exemplary embodiments of driving systems 250 are described in the PCT Publication No. WO 2019/112886, previously incorporated by reference.

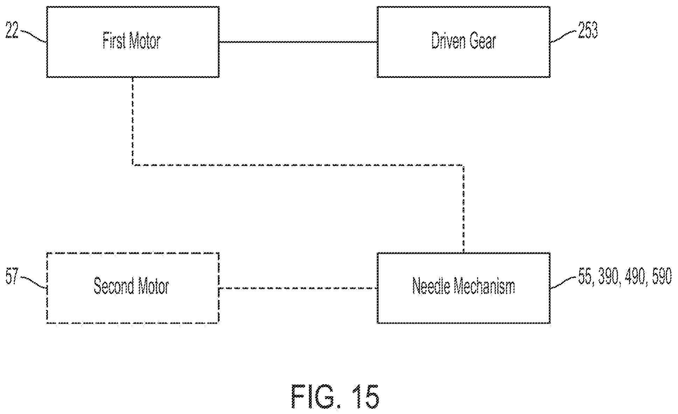

Referring to FIGS. 14-16, first motor 22 is operably coupled to driven gear 253 such that activation of first motor 22 actuates driven gear 253. Additionally, drug delivery device 1000 optionally comprises a second motor 57 (as shown in FIGS. 1-3), configured to actuate a needle mechanism, such as any of the needle mechanisms 55, 390, 490 described herein disclosed herein. In embodiments where drug delivery device does not comprise second motor 57, first motor 22 may be configured to actuate the needle mechanism(s).

Each of the first motor 22 and optional second motor 57 may be controlled by a motor controller 260. The term "logic" or "control logic" as used herein may include software and/or firmware executing on one or more programmable processors, application-specific integrated circuits (ASICs), field-programmable gate arrays (FPGAs), digital signal processors (DSPs), hardwired logic, or combinations thereof. Therefore, in accordance with the embodiments, various logic may be implemented in any appropriate fashion and would remain in accordance with the embodiments herein disclosed. Controller 260 may be included in drug delivery device 1000 or may be external. Controller 260 may include at least one processor 262 (e.g. microprocessor) that executes software and/or firmware stored in a memory 264 of controller 260. The software/firmware code contains instructions 265 that, when executed by the processor 262, cause controller 260 to perform the functions of the control algorithm described herein. Controller 260 may receive information from a plurality of system components and feed the information (e.g. medication data, patient data, drug delivery device data, needle assembly data) into the control algorithm which determines at least one drug delivery control parameter which may in part govern operation of first motor 22 and/or second motor 57. Controller 260 may include or be communicatively coupled to one or more interfaces to communicatively couple via one or more communication links to the drug delivery device 1000. Examples interfaces include wired and wireless signal transmitters and receivers. Example communication links include a wired communication link (e.g. a serial communication), a wireless communication link such as, for example, a short-range radio link, such as Bluetooth, IEEE 802.11, a proprietary wireless protocol, and/or the like. The term "communication link" may refer to an ability to communicate some type of information in at least one direction between at least two devices. The communication links may be a persistent communication link, an intermittent communication link, an ad-hoc communication link, and/or the like. Information may be transmitted via the communication links.

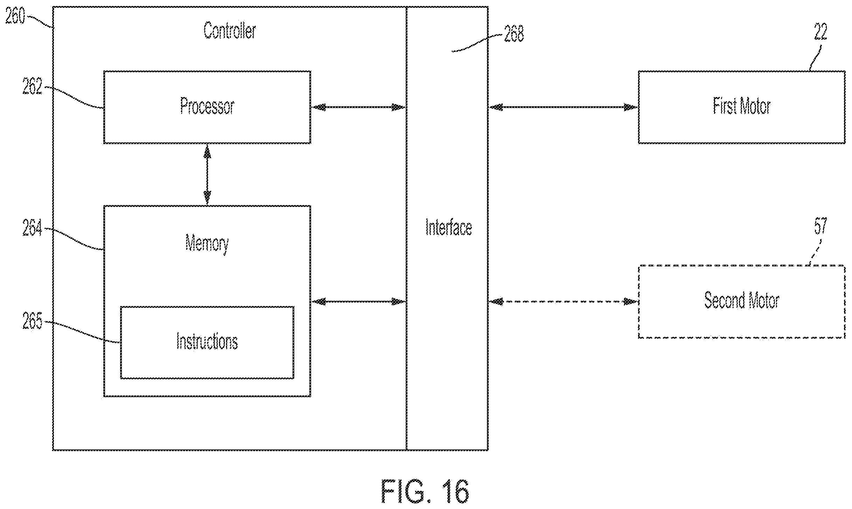

FIG. 16 shows the controller 260 including memory 264 and a processor 262 communicatively coupled to the one or more interfaces 268 and to each other. The memory 264 may include computer-readable storage media in the form of volatile and/or nonvolatile memory and may be removable, non-removable or a combination thereof. In embodiments, memory 264 stores executable instructions 265 (e.g. computer code, machine-useable instructions, and the like) for causing processor 262 to implement aspects of embodiments of system components discussed herein and/or to perform aspects of embodiments of methods and procedures discussed herein, including the control logic described in more detail below. The memory 264, processor 262, and interfaces 268 may be communicatively coupled by one or more busses. The data and/or information may be provided to controller 260 as acquired, on a predefined schedule, or queued inn memory and supplied to controller 260 when requested.

The logic of controller 260 may be configured to adjust the rate of actuation or the force of actuation provided by first motor 22 and/or second motor 57. For example, if the medication in cartridge 220 is viscous, the force applied by first motor 22 to drive stopper 224 may be increased. In a further example, the rate of actuation for the first or second motor 22, 57 may be adjusted to alter the rate of actuation for the needle mechanism. The needle mechanism may be actuated more slowly to improve patient comfort and reduce irritation. In some embodiments, the controller may alter the operating parameters of first and/or second motor 22, 57 based on received information/data (e.g. patient data, medication data, historical use data, dose data, drug delivery device data, needle assembly data, cartridge data). Additionally, controller 260 may be configured to alter the speed of motors 22, 57 over the course of activation such that motion of driving member 254 and/or the needle mechanism is not constant or nonlinear. For example, needle mechanism may be actuated such that the needles decelerate near the end of travel to prevent a hard stop. Controller 260 may also control timing of motor activation, for example activating first motor 22 before second motor 57. Controller 260 may also receive data based on needle position indicating the location of at least one needle in the needle assembly, and may alter the speed, force, or positioning of the at least one needle based on the position data.

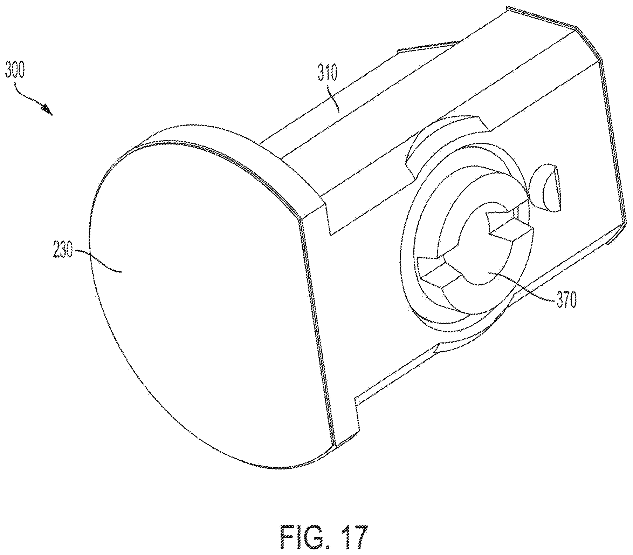

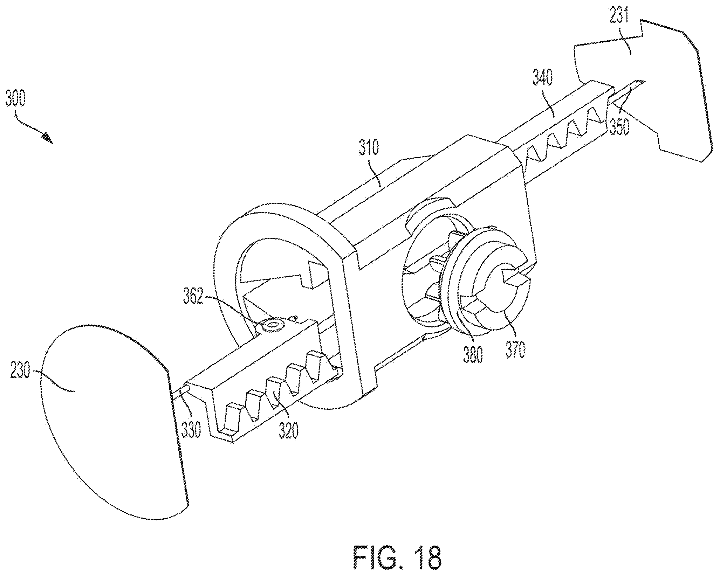

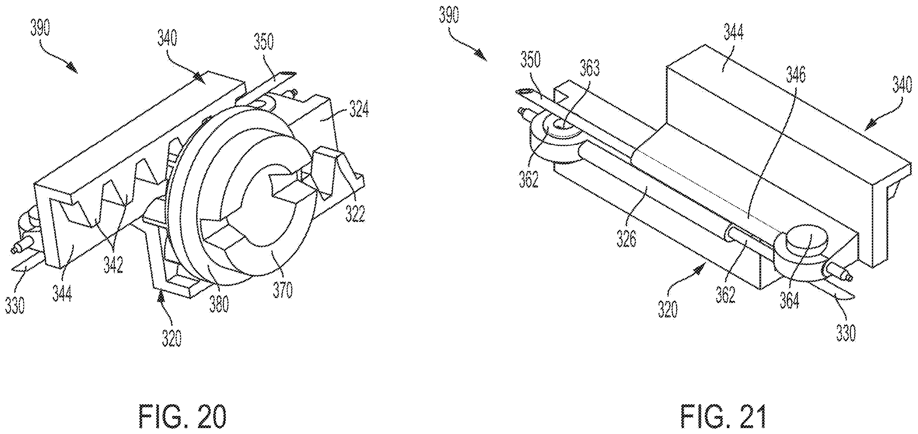

Referring now to FIGS. 17-19, an exemplary embodiment of a needle assembly 300 is shown. As discussed earlier, any of the illustrated needle assemblies or variations thereof may be used within drug delivery device 1000. Furthermore, the needle assemblies disclosed may be used separately from drug delivery device 1000, for example in chemical sampling instrumentation, or in other applications where a needle is used. Needle assembly 300 comprises an assembly housing 310, a first needle support 320 coupled to a first needle 330, a second needle support 340 coupled to a second needle 350, a first fluid coupler 362, a second fluid coupler 364, a driving member 370, and an O-ring 380. Furthermore, needle assembly 300 comprises an exterior seal 230 and an interior seal 231. Together, first needle support 320, first needle 330, second needle support 340, second needle 350, first fluid coupler 362, and second fluid coupler 364 comprise a needle mechanism 390 (FIG. 20). The exterior seal 230 and interior seal 231 are configured to seal the needle mechanism 390 within the needle assembly 300 before the needle assembly 300 is used, and also to help maintain sterile conditions within needle assembly 300.

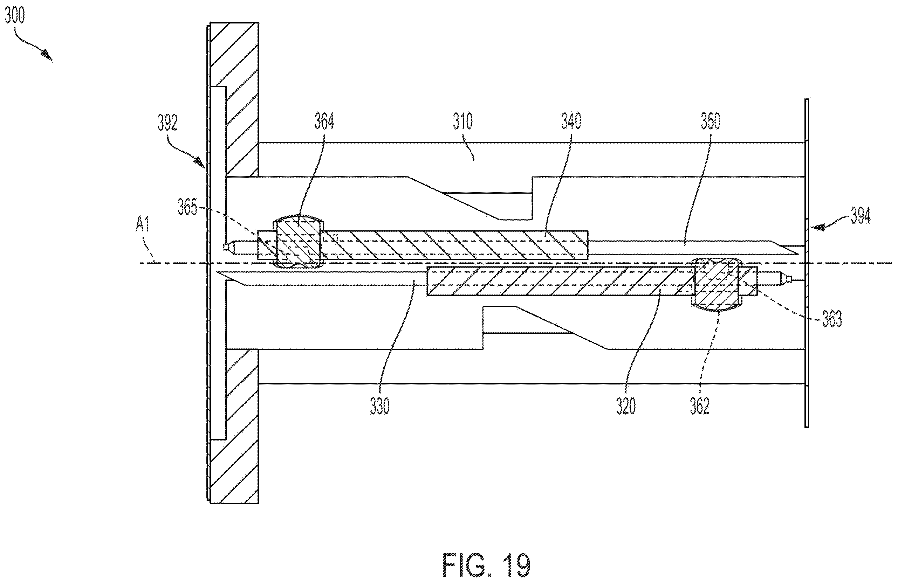

In the illustrated embodiment, needle assembly 300 is configured to interact with a patient's skin for delivery of the above-described medication. A first or distal end 392 of needle assembly 300 is positioned against the skin, and a second or proximal end 394 of needle assembly 300 interacts with cassette 200 (FIG. 12). The needle assembly 300 is configured to couple to cassette 200 either directly, or through needle assembly coupler 210. The first end 392 and/or the exterior seal 230 may comprise an adhesive to assist in positioning the needle assembly 300 against the patient's skin and may also comprise fasteners or adhesives to secure the needle assembly 300 to cassette 200. As shown in FIG. 19, the needle mechanism 390 is supported by the housing 310. In other embodiments, needle assembly 300 may comprise an additional internal housing supported by assembly housing 310 and configured to support needle mechanism 390. The internal housing may be separable from or otherwise movable in relation to the assembly housing 310.

Referring to FIG. 19, the needle mechanism 390 is configured to fit entirely within needle assembly 300 when in a first configuration. FIGS. 19, 20, and 21 show various views of needle mechanism 390 in the first configuration. In the first configuration, first needle 330 and second needle 350 are approximately aligned parallel to one another and parallel to central axis A1. As shown, first needle 330 and second needle 350 are pointed in approximately opposite directions along axis A1. First needle 330 is pointed towards first end 392 of needle assembly 300, and second needle 350 is pointed towards second end 394 of needle assembly 300. As will be described later, first needle 330 and second needle 350 are configured to move generally parallel to axis A1 when the needle assembly 300 is activated.

Referring to FIGS. 20-21, an exemplary embodiment of needle mechanism 390 is shown. First needle support 320 and second needle support 340 are positioned generally along a common plane and are slidably coupled to one another through driving member 370. A first side of first and second needle supports 320, 340 is configured to interact with driving member 370, and a second side of first and second needle supports 320, 340 is configured to interact with and support first and second needles 330, 350. In the illustrated embodiment, first and second needle supports 320, 340 are generally rectangular in shape and are configured to fit within needle assembly 300. In other embodiments, first and second needle supports 320, 340 may be any shape to accommodate motion of needles 330, 350 and fit within needle assembly 300. In the illustrated embodiment, needle mechanism 390 comprises a rack and pinion configuration, wherein each of first needle support 320 and second needle support 340 is a rack, and driving member 370 includes a pinion gear. First needle support 320 comprises a support body 324, support engagement features 322, and a needle retainer 326. The needle retainer 326 is configured to couple with first needle 330 330 by inserting at least of portion of first needle 330 into needle retainer 326, thereby coupling first needle 330 to first needle support 320. Similarly, second needle support 340 comprises a support body 344, support engagement features 342, and a needle retainer 346, and the second needle 350 is coupled to second needle support 340 through needle retainer 346. In other embodiments, one or both of first needle support 320 and second needle support 340 may additionally comprise a needle guard (not shown), which may function to protect the needle and/or to provide additional strength to the needle. The needle guard may be a cylindrical housing with a bore extending therein, through which the respective needle 330, 350 extends.

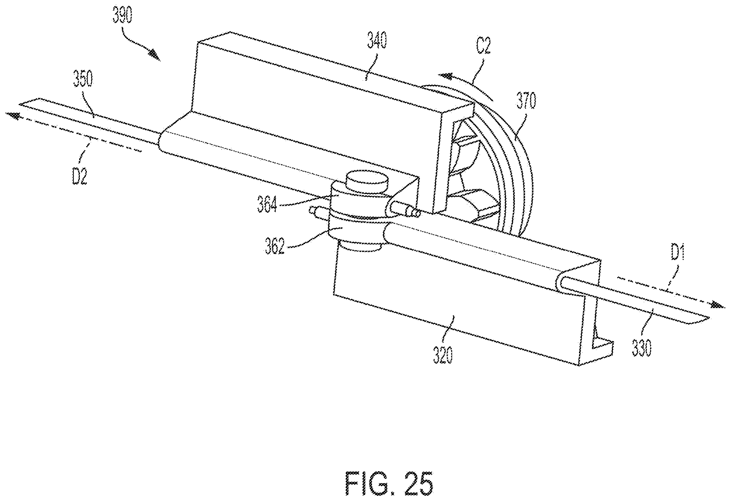

Referring to FIGS. 19-21, first needle support 320 further comprises the first fluid coupler 362 and second needle support 340 further comprises the second fluid coupler 364. The first fluid coupler 362 and second fluid coupler 364 comprise an upward-facing first bore 363 and a downward-facing second bore 365, respectively. First fluid coupler 362 is fluidly coupled to first needle 330, and second fluid coupler 364 is fluidly coupled to second needle 350 through the first bore 363 and second bore 365, respectively. The fluid couplers 362 and 364 are configured such that they are fluidly disconnected when separated from one another in the first configuration (See FIGS. 19, 20, and 24) and fluidly coupled when aligned with one another in the second configuration (See FIG. 25). In this second configuration, a fluid may flow from the second needle 350, downward through the second fluid coupler 364, into first fluid coupler 362, and out of first needle 330. A fluid may also travel in the opposite direction from first needle 330 to second needle 350. In one embodiment, first fluid coupler 362 and second fluid coupler 364 comprise a sealing element around the surface near first bore 363 and second bore 365, respectively. The sealing element may be an O-ring, an engagement feature configured to form a seal or otherwise couple first fluid coupler 362 to second fluid coupler 364 such as protrusions or grooves, an adhesive, or another surface feature configured to prevent fluid from flowing out of the fluid path formed by first needle 330, first fluid coupler 362, second fluid coupler 364, and second needle 350. Furthermore, each of first fluid coupler 362 and second fluid coupler 364 may comprise a stopping feature configured to prevent the flow a fluid when the fluid couplers 362, 364 are not aligned. In an exemplary embodiment, the stopping feature is a spring-loaded stopper.

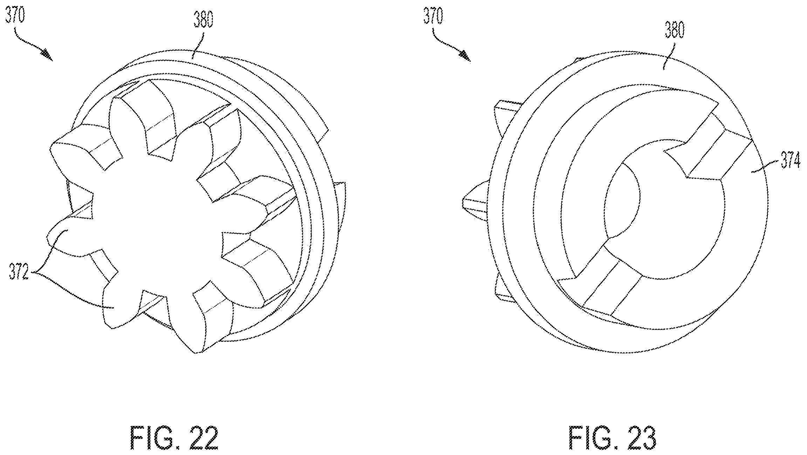

As shown best in FIGS. 22 and 23, driving member 370 comprises driving engagement features 372, and an actuating member 374. The O-ring 380 may be provided on driving member 370 in order to provide a better seal between driving member 370 and assembly housing 310 (FIG. 17), or to allow driving member 370 to rotate more easily within assembly housing 310. The driving engagement features 372 are configured to interact with the support engagement features 322 and 342 (FIG. 20). In the illustrated example, driving engagement features 372 and support engagement features 322 and 342 are teeth or protrusions configured to mesh together in a rack and pinion mechanism. In other embodiments, any of the engagement features within needle mechanism 390 may be other features configured to interact with one another such as protrusions and recessions, screws and threads, zipper type features, or any other complimentary engagement features. In another embodiment, the driving member 370 may be a drive screw, and the needle mechanism 390 may comprise a thread to engage with the drive screw. Furthermore, in the illustrated example, actuating member 374 is configured to be actuated by a rotation of a flat object, such as a flat-head screwdriver. In other embodiments, actuating member 374 may be actuated by an electric motor, a torsional spring, or human interaction such as rotation of a knob. In one exemplary embodiment, both the driving system 250 (FIGS. 11 and 12) and driving member 370 are each independently driven by an electric motor. One electric motor may control the movement of both driving system 250 and driving member 370, or they may each have their own respective actuating motors.

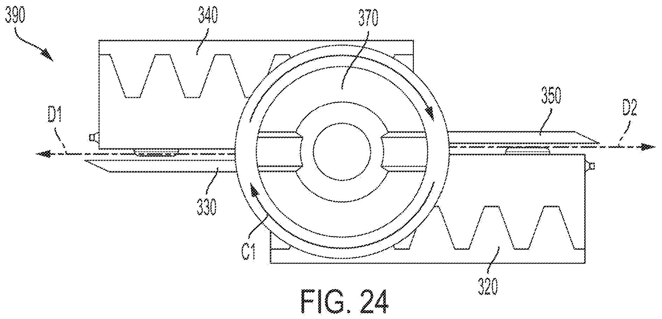

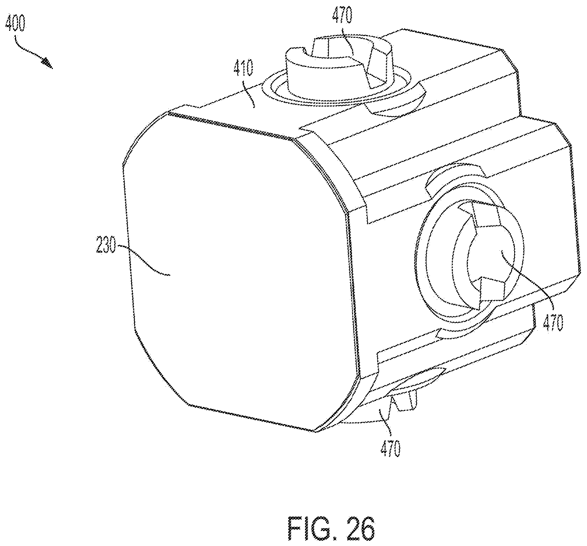

Referring now to FIGS. 24-26, the needle mechanism 390 is configured to allow for movement of first needle support 320 and second needle support 340 upon activation of driving member 370. Needle mechanism 390 is movable between a first, retracted configuration before use and/or after use, as shown in FIG. 24, and a second, extended configuration during use, as shown in FIG. 26.

In the first, retracted configuration of FIG. 24, both first needle 330 and second needle 350 are axially positioned entirely within assembly housing 310. Upon rotation of driving member 370 in a direction C1, first needle support 320 and first needle 330 are driven in a direction D1, and second needle support 340 and second needle 350 are driven in a direction D2 until the rotation of driving member 370 stops once the needle mechanism 390 is in the second configuration. In the illustrated embodiment, D1 and D2 are approximately parallel and are generally opposite of one another. Furthermore, they are approximately parallel to axis A1 (See FIG. 19). In other embodiments, D1 and D2 may be angled relative to one another, and may not be approximately parallel to A1.

In the second, extended configuration of FIG. 25, first needle 330 extends axially beyond assembly housing 310 and passes through outer seal 230 and into whatever surface needle assembly 300 is positioned against. Additionally, second needle 350 extends axially beyond assembly housing 310 and passes through interior seal 231 and septum 226 into fluid housing 222. In the second configuration, first needle 330 is fluidly coupled to cartridge 220, so the fluid within fluid housing 222 is capable of flowing through second needle 350, second fluid coupler 364, first fluid coupler 362, and first needle 330 into whatever body or surface first needle 330 has pierced, as described above. In an exemplary embodiment, the driving system 250 (FIGS. 12 and 14) is activated after needle mechanism 390 is in the second configuration, and the fluid within cartridge 220 is driven out and into the body or surface pierced by first needle 330. In another embodiment, a number of components of needle mechanism 390, and/or the internal housing 312 comprise a stopping feature to physically stop the movement of first needle support 320 and second needle support 340 once needle mechanism 390 reaches the second configuration. Such a stopping feature may be a blocking member, a protrusion, a detent, or an absence of engagement members within a portion of the needle mechanism 390.

From the second configuration, needle mechanism 390 is configured to be movable back to the first configuration through reverse activation of driving member 370 as well. As shown in FIG. 25, driving member 370 may be rotated in a direction C2, which is generally opposite to C1, in order to drive first needle support 320 in the direction D2, and second needle support 340 in the direction D1. In an exemplary embodiment, needle mechanism 390 is moved from the second configuration to the first configuration after the fluid has been driven out of cartridge 220 and through needle mechanism 390. In an embodiment where drug delivery device 1000 is being used to deliver a medication to a patient, extending the first needle 330 from needle assembly 300, and then retracting the first needle 330 back into the needle assembly 300 after injection would allow the patient to be injected with a medication without needing to directly observe or handle the first needle 330.

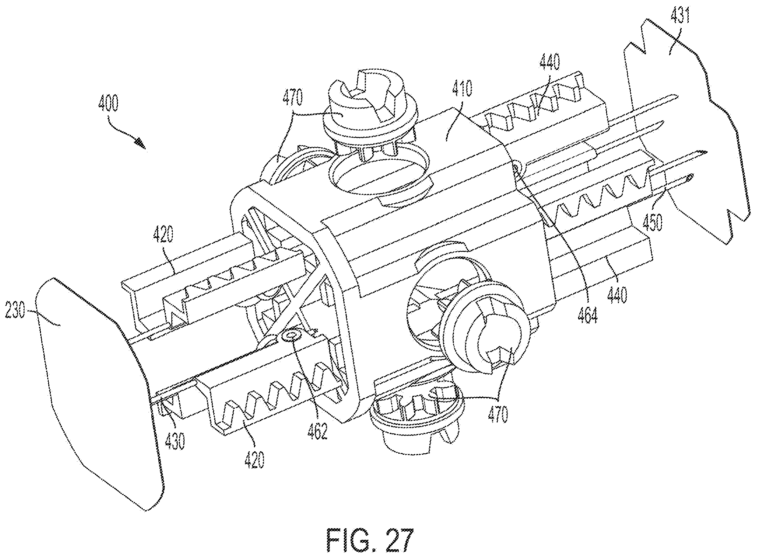

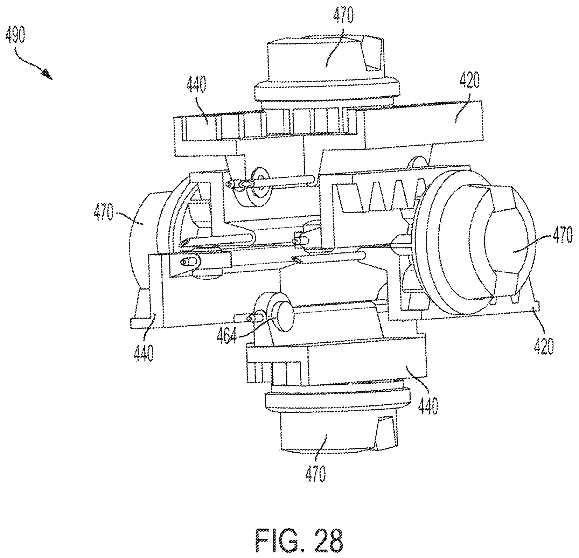

Referring now to FIGS. 26-28, another exemplary embodiment of a needle assembly 400 is shown. Needle assembly 400 is similar to needle assembly 300, but instead of a single needle mechanism, needle assembly 400 comprises a plurality of needle mechanisms 490. In the illustrated embodiment, four needle mechanisms 490 are shown, but any number of needle mechanisms 490 may be used. Each of the needle mechanisms 490 comprise a first needle support 420 coupled to a first needle 430, a second needle support 440 coupled to a second needle 450, a first fluid coupler 462, a second fluid coupler 464, a driving member 470, and an O-ring 480. Each needle mechanism 490 within needle assembly 400 operates essentially the same as the individual needle mechanism 390 within needle assembly 300, as described above. In an exemplary embodiment, only one of the needle mechanisms 490 is activated at a time, and so only one of the needle mechanisms 490 is in the second, extended configuration when needle assembly 400 is used. Furthermore, in the exemplary embodiment, each of the needle mechanisms 490 are sterile before use.

In the illustrated embodiment, needle assembly 400 additionally comprises a housing 410 and an interior seal 431 similar to interior seal 231. In another embodiment, needle assembly 400 may comprise a plurality of interior seals. The plurality of interior seals may be configured to function similarly to interior seal 431 but accomplished with a plurality of smaller seals instead of one larger seal. The interior seals may be positioned to cover holes in housing 410 corresponding to a position of a needle mechanism 490 within the needle assembly 400. When the cassette 200 is coupled to needle assembly 400, the cassette 200 may comprise a single cartridge 220 (FIGS. 12-13) to align with a single interior seal 431 or may comprise a number of cartridges 220 to align with each of a plurality of interior seals.

Needle assembly 400 may also comprise a rotating assembly (not shown). The rotating assembly may rotate the entirety of needle assembly 400, or just the needle mechanisms 490 within needle assembly 400 in order to align one of the needle mechanisms 490 with the cartridge 220. The needle mechanism 490 that is aligned with the cartridge 220 may then move from the first configuration to the second configuration (as described with respect to needle mechanism 390) to cause the second needle 450 to pierce the interior seal 431 and septum 226, and first needle 430 to pierce exterior seal 230 and the surface or skin that needle assembly 400 is positioned against. The needle mechanism 490 may then be moved back to the second configuration (e.g. after drug delivery as described with respect to needle assembly 300), and then the rotating assembly can rotate another needle mechanism 490 to align with cartridge 220. The new needle mechanism 490 to be aligned with cartridge 220 may then be activated to pierce the same cartridge 220 again, or a new cartridge 220 may be used. In an exemplary embodiment, the rotating assembly is a gear to be driven by another gear, a screw, a rack, or another gear engaging feature. Additionally, the rotating assembly may be driven by an electric motor. In another embodiment, at least one of the rotating assembly, the housing 410, and the needle mechanisms 490 have an engagement feature to stop the rotation assembly in the correctly aligned position. Examples of engagement features include indents, grooves, latches, protrusions, detent-and-ball mechanisms, and other locking features. Having multiple needle mechanisms 490 within needle assembly 400 allows for multiple doses of a medication to be delivered with the same needle assembly 400, such that a different, sterile needle 430 is used for each injection. Additionally, different medications may be injected in through the same needle assembly 400, but through different needle mechanisms 490. In some embodiments, drug delivery device 1000 comprises a counter or a needle counter (not shown) that tracks how many and/or which needles have been used. For example, a needle index may increase after a needle mechanism 490 has been extended from the first configuration to the second configuration, or when the needle assembly 400 rotates. Drug delivery device 1000 may indicate to a user when each of the available needles has been used.

Multiple embodiments of needle assemblies and needle mechanisms have been disclosed. It should be understood that the features and configurations of each embodiment disclosed may be applied to any other embodiment disclosed, either alone or in combination with other features and configurations. Many of the features discussed are attributed to drug delivery device 1000, but they may also be applied to any other embodiment of drug delivery device 11, 12, 13, 126 described herein, or any modifications thereof.

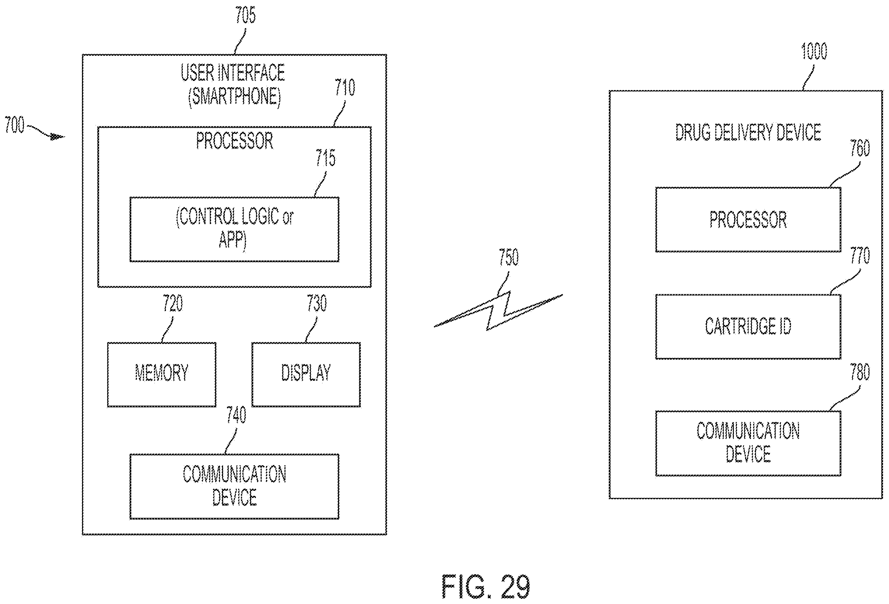

Referring now to FIG. 29, a computing device 700 may be used to communicate with and/or operate any of the drug delivery devices 11, 12, 13, 126, 1000 described herein, illustratively drug delivery device 1000. Computing device 700 illustratively includes a mobile device, such as a smartphone. Alternatively, any suitable computing device may be used, including but not limited to a laptop, desktop, tablet, or server computer, for example.

The computing device 700 includes at least one processor 710 that executes software and/or firmware stored in memory 720 of device 700. The software/firmware code contains instructions that, when executed by processor 710, causes device 700 to perform the functions described herein. The at least one processor 710 illustratively includes control logic and/or an application 715 operative to activate drug delivery device 1000. Memory 720 is any suitable computer readable medium that is accessible by processor 710. Memory 720 may be a single storage device or multiple storage devices, may be located internally or externally to processor 710, and may include both volatile and non-volatile media. Exemplary memory 720 includes random-access memory (RAM), read-only memory (ROM), electrically erasable programmable ROM (EEPROM), flash memory, a magnetic storage device, optical disk storage, or any other suitable medium which is configured to store data and which is accessible by processor 710.

Computing device 700 includes a user interface 705 in communication with processor 710 and operative to provide user input data to the system and to receive and display data, information, and prompts generated by the system. User interface 705 includes at least one input device for receiving user input and providing the user input to the system. In the illustrated embodiment, user interface 705 is a graphical user interface (GUI) including a touchscreen display operative to display data and receive user inputs. The touchscreen display allows the user to interact with presented information, menus, buttons, and other data to receive information from the system and to provide user input into the system. Alternatively, a keyboard, keypad, microphone, mouse pointer, or other suitable user input device may be provided.

Computing device communicates with drug delivery device 1000 through signal 750. Signal 750 may be a wireless or wired signal. Drug delivery device 1000 may comprise a processor 760 similar to processor 710, a cartridge ID 770, and/or a communication device 780. Communication device 780 may send or receive a signal 750 to/from communication device 740, or to/from other components of drug delivery device 1000 (e.g. cartridge ID 770). Cartridge ID 770 may be any sort of mechanism or device that provides data about a component of drug delivery device 1000. For example, cartridge ID 770 may be a chip or RFID indicator on cartridge 220 (FIG. 12) that provides data regarding the type of liquid or medication within cartridge 220 (e.g. specific medication, viscosity, volume, dosage, injection scheduling, etc.). Other components, such as a needle assembly or motor, may include an ID to communicate other data related to drug delivery device 1000 (e.g. needle assembly type, needle length/positioning, patient information, historical treatment information, motor type, motor characteristics, etc.).

In some embodiments, drug delivery device 1000 may comprise an indicator (not shown) that provides some sort of indication that the medication has been delivered to the patient. Such an indication may be an end of dose indication. The indicator may comprise, for example, a light (e.g. an LED), a visual display such as a screen, a vibration, a sound, the sending of a signal, an indication on a separate computing device (e.g. a smartphone or computer as discussed above), a mechanical visual indicator (e.g. a window in the device housing to show barrel movement), or any combination thereof. The indicator may also indicate to a user any other information about the operation of drug delivery device 1000 including, but not limited to, whether or not a cartridge is inserted, whether a cartridge is inserted properly, device power information (e.g. whether the device is on/off, battery level), patient information, any information that may be provided by ID's discussed above, or any combination thereof.

In some embodiments, drug delivery device 1000 may comprise a number of sensors (not shown) that sense information related to the device. In an exemplary embodiment, drug delivery device 1000 comprises a skin sensor which senses whether the device is properly positioned against a patient's skin. In some embodiments, the drug delivery device 1000 may not actuate a needle assembly or deliver a medication if the skin sensor does not indicate that the device is positioned properly. Other examples of optional sensors include, but are not limited to, a medication level sensor, a pressure sensor, accelerometers, a force/thrust sensor, a position sensor for elements of the driving system, cartridge, housing, and/or needle assembly, a pH sensor, or any combination thereof.

The terms "first", "second", "third" and the like, whether used in the description or in the claims, are provided for distinguishing between similar elements and not necessarily for describing a sequential or chronological order. It is to be understood that the terms so used are interchangeable under appropriate circumstances (unless clearly disclosed otherwise) and that the embodiments of the disclosure described herein are capable of operation in other sequences and/or arrangements than are described or illustrated herein.

While this invention has been described as having exemplary designs, the present invention can be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

Various aspects are described in this disclosure, which include, but are not limited to, the following aspects:
1. A drug delivery device comprising: a cassette; a cartridge within the cassette configured to hold a medication; a needle assembly coupled to the cassette comprising: a housing with a first end and a second end configured to couple to the cassette; a needle mechanism supported by the housing, comprising a first needle and a second needle, and movable from a first configuration to a second configuration, wherein in the first configuration the needle mechanism is positioned fully within the housing and in the second configuration the first needle extends beyond the first end and the second needle extends beyond the second end; and a first fluid coupler and a second fluid coupler within the needle mechanism and fluidly coupled to the first needle and the second needle respectively, wherein the first and second fluid couplers fluidly disconnect the first and second needles when the needle mechanism is in the first configuration and fluidly couple the first and second needles when the needle mechanism is in the second configuration.
2. The drug delivery device of aspect 1, wherein the cassette is decouplable from the needle assembly.
3. The drug delivery device of any one of aspects 1-2, wherein the needle mechanism further comprises a first needle support and a second needle support configured to support the first needle and the second needle respectively.
4. The drug delivery device of aspect 3, wherein the needle mechanism further comprises a driving member configured to interact with the first and second needle supports.
5. The drug delivery device of aspect 4, wherein the driving member drives the first and second needle supports to move the needle mechanism from the first configuration to the second configuration.
6. The drug delivery device of aspect 5, wherein the driving member is additionally configured to drive the first and second needle supports to move the needle mechanism from the second configuration back to the first configuration.
7. The drug delivery device of aspect 4, wherein the driving member comprises a pinion gear and each of the first and second needle supports comprise racks.
8. The drug delivery device of any one of aspects 1-7, wherein the needle mechanism is additionally movable from the second configuration back to the first configuration.
9. The drug delivery device of any one of aspects 1-8, further comprising a driving mechanism coupled to the cassette and configured to drive the drug from the cartridge through the needle assembly.
10. The drug delivery device of any one of aspects 1-9, wherein the first end of the housing comprises a seal and the cartridge comprises a septum proximate the second end of the housing, and when moving from the first configuration to the second configuration the first needle pierces the seal and the second needle pierces the septum.
11. The drug delivery device of any one of aspects 1-10, wherein the needle mechanism is driven by a variable speed motor.
12. The drug delivery device of aspect 11, wherein the variable speed motor is configured to alter a motor speed based on at least one sensor within the drug delivery device.
13. A method of operation of a device comprising the steps of: positioning the device, the delivery device comprising a cassette, a drug cartridge within the cassette, and movable needle assembly coupled to the cartridge; activating a driving member of the needle assembly, the driving member moving a first needle from the needle assembly in a first direction from a retracted configuration to an axially extended configuration , and moving a second needle from the needle assembly in a second direction towards the drug cartridge; fluidly coupling the first needle and the second needle; activating a delivery mechanism to drive a medication from the drug cartridge through the needle assembly; and reactivating the driving member to move the first needle in the second direction from the axially extended configuration to the retracted configuration, and to move the second needle in the first direction away from the septum.
14. The method of aspect 13, further comprising the steps of decoupling the cassette from the needle assembly and coupling a new cassette onto the needle assembly.
15. The method of any one of aspects 13-14, further comprising the step of piercing a septum of the drug cartridge with the second needle after moving the second needle towards the drug cartridge.
16. The method of any one of aspects 13-15, wherein the driving member and the delivery mechanism are each activated by at least one electric motor.
17. The method of any one of aspects 13-16, further comprising the step of rotating at least a portion of the needle assembly to align a third needle with the skin and a fourth needle with the drug cartridge.
18. The method of any one of aspects 13-17, further comprising the step of indicating to a user that the medication has been driven from the drug cartridge.
19. A needle assembly comprising a housing configured to interface with a surface; a first needle supported by a first needle support positioned at least partially within the housing; a second needle supported by a second needle support positioned at least partially within the housing; a driving member supported by the housing and configured to engage with the first needle support and the second needle support and to move the first and second needle supports relative to the housing; a first fluid coupler in fluid communication with the first needle; and a second fluid coupler in fluid communication with the second needle, wherein the first and second fluid couplers fluidly couple the first and second needles when the first and second fluid couplers are aligned.
20. The needle assembly of aspect 19, wherein the first needle and the second needle are oriented in approximately opposite directions along a central axis.
21. The needle assembly of aspect 20, wherein the driving member is configured to move the first needle in a first direction, and to move the second needle in a second direction, the second direction being approximately opposite the first direction.
22. The needle assembly of any one of aspects 19-21, further comprising a first seal at a first end of the housing, and a second seal at the second end of the housing.
23. The needle assembly of aspect 22, wherein the first needle is configured to pierce the first seal, and the second needle is configured to pierce the second seal.
24. A needle assembly comprising: a housing with a first end and a second end; a plurality of needle mechanisms supported by the housing, each of the needle mechanisms comprising: a first needle support configured to move in a first direction; a second needle support configured to move in a second direction; a first fluid coupler coupled to the first needle support; a second fluid coupler coupled to the first needle support, wherein the first fluid coupler and the second fluid coupler provide fluid communication between the first needle and the second needle when the first and second fluid couplers are aligned; and a rotating assembly configured to rotate the needle mechanisms within the housing.

The claims of the parent application are reproduced immediately below as clauses. These clauses define preferred embodiments. The applicant reserves the right to pursue protection for subject-matter contained in the parent application as filed, either in the present divisional application or in a further application divided from the present divisional application.
1. A drug delivery device comprising:
   a cassette;
   a cartridge within the cassette configured to hold a medication;
   a needle assembly coupled to the cassette comprising:
      a housing with a first end and a second end configured to couple to the cassette;
      a needle mechanism supported by the housing, comprising a first needle and a second needle, and movable from a first configuration to a second configuration, wherein in the first configuration the needle mechanism is positioned fully within the housing and in the second configuration the first needle extends beyond the first end and the second needle extends beyond the second end; and
      a first fluid coupler and a second fluid coupler within the needle mechanism and fluidly coupled to the first needle and the second needle respectively, wherein the first and second fluid couplers fluidly disconnect the first and second needles when the needle mechanism is in the first configuration and fluidly couple the first and second needles when the needle mechanism is in the second configuration.
2. The drug delivery device of clause 1, wherein the cassette is decouplable from the needle assembly.
3. The drug delivery device of any one of clauses 1-2, wherein the needle mechanism further comprises a first needle support and a second needle support configured to support the first needle and the second needle respectively.
4. The drug delivery device of clause 3, wherein the needle mechanism further comprises a driving member configured to interact with the first and second needle supports.
5. The drug delivery device of clause 4, wherein the driving member drives the first and second needle supports to move the needle mechanism from the first configuration to the second configuration.
6. The drug delivery device of clause 5, wherein the driving member is additionally configured to drive the first and second needle supports to move the needle mechanism from the second configuration back to the first configuration.
7. The drug delivery device of clause 4, wherein the driving member comprises a pinion gear and each of the first and second needle supports comprise racks.
8. The drug delivery device of any one of clauses 1-7, wherein the needle mechanism is additionally movable from the second configuration back to the first configuration.
9. The drug delivery device of any one of clauses 1-8, further comprising a driving mechanism coupled to the cassette and configured to drive the drug from the cartridge through the needle assembly.
10. The drug delivery device of any one of clauses 1-9, wherein the first end of the housing comprises a seal and the cartridge comprises a septum proximate the second end of the housing, and when moving from the first configuration to the second configuration the first needle pierces the seal and the second needle pierces the septum.
11. The drug delivery device of any one of clauses 1-10, wherein the needle mechanism is driven by a variable speed motor.
12. The drug delivery device of clause 11, wherein the variable speed motor is configured to alter a motor speed based on at least one sensor within the drug delivery device.
13. A method of operation of a device comprising the steps of:
   positioning the device, the delivery device comprising a cassette, a drug cartridge within the cassette, and movable needle assembly coupled to the cartridge;
   activating a driving member of the needle assembly, the driving member moving a first needle from the needle assembly in a first direction from a retracted configuration to an axially extended configuration , and moving a second needle from the needle assembly in a second direction towards the drug cartridge;
   fluidly coupling the first needle and the second needle;
   activating a delivery mechanism to drive a medication from the drug cartridge through the needle assembly; and
   reactivating the driving member to move the first needle in the second direction from the axially extended configuration to the retracted configuration, and to move the second needle in the first direction away from the septum.
14. The method of clause 13, further comprising the steps of decoupling the cassette from the needle assembly and coupling a new cassette onto the needle assembly.
15. The method of any one of clauses 13-14, further comprising the step of piercing a septum of the drug cartridge with the second needle after moving the second needle towards the drug cartridge.
16. The method of any one of clauses 13-15, wherein the driving member and the delivery mechanism are each activated by at least one electric motor.
17. The method of any one of clauses 13-16, further comprising the step of rotating at least a portion of the needle assembly to align a third needle with the skin and a fourth needle with the drug cartridge.
18. The method of any one of clauses 13-17, further comprising the step of indicating to a user that the medication has been driven from the drug cartridge.
19. A needle assembly comprising
   a housing configured to interface with a surface;
   a first needle supported by a first needle support positioned at least partially within the housing;
   a second needle supported by a second needle support positioned at least partially within the housing;
   a driving member supported by the housing and configured to engage with the first needle support and the second needle support and to move the first and second needle supports relative to the housing;
   a first fluid coupler in fluid communication with the first needle; and
   a second fluid coupler in fluid communication with the second needle, wherein the first and second fluid couplers fluidly couple the first and second needles when the first and second fluid couplers are aligned.
20. The needle assembly of clause 19, wherein the first needle and the second needle are oriented in approximately opposite directions along a central axis.
21. The needle assembly of clause 20, wherein the driving member is configured to move the first needle in a first direction, and to move the second needle in a second direction, the second direction being approximately opposite the first direction.
22. The needle assembly of any one of clauses 19-21, further comprising a first seal at a first end of the housing, and a second seal at the second end of the housing.
23. The needle assembly of clause 22, wherein the first needle is configured to pierce the first seal, and the second needle is configured to pierce the second seal.
24. A needle assembly comprising:
   a housing with a first end and a second end;
   a plurality of needle mechanisms supported by the housing, each of the needle mechanisms comprising:
      a first needle support configured to move in a first direction; a second needle support configured to move in a second direction;
      a first fluid coupler coupled to the first needle support;
      a second fluid coupler coupled to the first needle support, wherein the first fluid coupler and the second fluid coupler provide fluid communication between the first needle and the second needle when the first and second fluid couplers are aligned; and
   a rotating assembly configured to rotate the needle mechanisms within the housing.

## Claims

1. A needle assembly comprising
a housing configured to interface with a surface;
a first needle supported by a first needle support positioned at least partially within the housing;
a second needle supported by a second needle support positioned at least partially within the housing;
a driving member supported by the housing and configured to engage with the first needle support and the second needle support and to move the first and second needle supports relative to the housing;
a first fluid coupler in fluid communication with the first needle; and
a second fluid coupler in fluid communication with the second needle,
wherein the first and second fluid couplers fluidly couple the first and second needles when the first and second fluid couplers are aligned.

2. The needle assembly of claim 1, wherein the first needle and the second needle are oriented in approximately opposite directions along a central axis.

3. The needle assembly of claim 2, wherein the driving member is configured to move the first needle in a first direction, and to move the second needle in a second direction, the second direction being approximately opposite the first direction.

4. The needle assembly of claim 3, wherein the driving member is configured to move the first needle in the second direction, and to move the second needle in the first direction.

5. The needle assembly of any one of claims 1-4, further comprising a first seal at a first end of the housing, and a second seal at the second end of the housing.

6. The needle assembly of claim 5, wherein the first needle is configured to pierce the first seal, and the second needle is configured to pierce the second seal.

7. The needle assembly of claim 1, wherein the driving member comprises a pinion gear and each of the first and second needle supports comprise racks.

8. The needle assembly of any one of claims 1-7, wherein each of the first fluid coupler (362) and the second fluid coupler (364) comprises a bore (363, 365), respectively.

9. The needle assembly of claim 8, wherein each of the first and second fluid couplers comprises a sealing element around a surface near the bore.

10. The needle assembly of any one of claims 1-7, wherein the driving member is driven by a motor.
